# EUROPÄISCHE PATENTANMELDUNG

(11) **EP 4 046 626 A1**
(43) Veröffentlichungstag der Anmeldung: **24.08.2022**
(21) Anmeldenummer: 21158312.5
(22) Anmeldetag: 21.02.2021
(51) Int. Cl.: A61K 9/00, A61K 9/08, A61K 31/519, A61K 47/02, A61K 47/10, A61K 47/20, A61K 47/26

(54) **VERWENDUNG EINER PEMETREXED-KONZENTRATLÖSUNG**

(71) Anmelder: STADA Arzneimittel AG, 61118 Bad Vilbel (DE)
(72) Erfinder: SCHRIDDE, Edgar., 30855 Langenhagen (DE)
(74) Vertreter: Kernebeck, Thomas

(57) **Zusammenfassung**

Die vorliegende Erfindung betrifft die Verwendung einer flüssigen pharmazeutische Konzentratlösung, umfassend
- ein Lösungsmittel;
- Pemetrexed oder ein pharmazeutisch annehmbares Salz davon; sowie
- Acetylcystein und/oder 2-Mercaptoethansulfonat-Natrium als ein erstes Antioxidationsmittel.

Um eine Verwendung besagter Konzentratlösung bereitzustellen, die eine relativ hohen Gebrauchswertigkeit für den Anwender ermöglicht, wird vorgeschlagen, dass die Konzentratlösung zur Herstellung einer Infusionslösung mittels einer Verdünnungslösung verwendet wird, wobei die Infusionslösung nach ihrer Herstellung für einen Zeitraum von länger als 72 Stunden stabil ist.

## Beschreibung

Die vorliegende Erfindung betrifft die Verwendung einer flüssigen pharmazeutische Pemetrexed-Konzentratlösung, umfassend
- ein Lösungsmittel;
- Pemetrexed oder ein pharmazeutisch annehmbares Salz davon; sowie
- Acetylcystein und/oder 2-Mercaptoethansulfonat-Natrium als ein erstes Antioxidationsmittel.

Pemetrexed ist der internationale Freiname (INN (International Nonproprietary Name)) für N-[4-[2-(2-Amino-4,7-dihydro-4-oxo-1H-pyrrolo[2,3-d]pyrimidin-5-yl)ethyl]-benzoyl]-L-glutaminsäure. Pemetrexed weist die folgende Strukturformel auf:

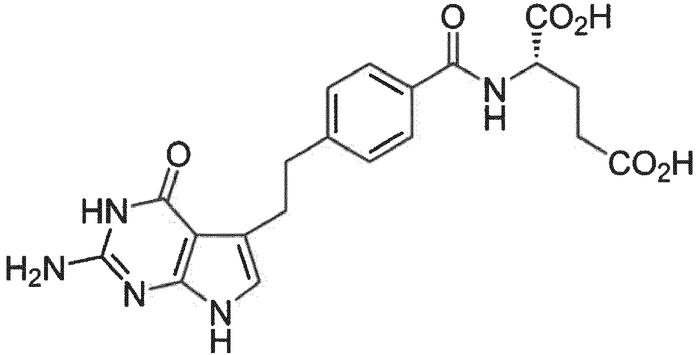

Pemetrexed ist ein Arzneistoff aus der Gruppe der Folsäureantagonisten, der insbesondere zur Behandlung zweier Lungenkrebsarten angewendet wird:
a) malignes Pleuramesotheliom (einer Krebsart des Lungenfells, die in der Regel durch eine Asbestexposition ausgelöst wird), wo es zusammen mit Cisplatin bei Patienten angewendet wird, die zuvor keine Chemotherapie erhalten haben und bei denen der Krebs nicht durch eine Operation entfernt werden kann.
b) fortgeschrittener "nicht-kleinzelliger" Lungenkrebs vom "nicht-squamösen" Typ, wo es entweder zusammen mit Cisplatin bei zuvor unbehandelten Patienten oder allein bei Patienten angewendet wird, die zuvor Arzneimittel gegen Krebs erhalten haben. Es kann auch als Erhaltungstherapie bei Patienten angewendet werden, die eine Chemotherapie auf Platinbasis erhalten haben.

Die Wirkung von Pemetrexed beruht auf der Blockierung sowohl der Thymidilat-Synthase als auch der Dihydrofolat-Reduktase und der Glycinamidribonucleotidformyl-Transferase, wodurch die Folat-abhängige Biosynthese von Thymidin und Purinnucleotiden gehemmt wird (vgl. Mutschler Arzneimittelwirkungen, Lehrbuch der Pharmakologie und Toxikologie, 9. Auflage, Wissenschaftliche Verlagsgesellschaft mbH Stuttgart, 2008, S. 919-920; ISBN 978-3-8047-1952-1).

Pemetrexed wird unter der Markenbezeichnung Alimta^{®} in der Darreichungsform eines Lyophilisates in den Wirkstärken 100 mg und 500 mg vertrieben. Für die Verabreichung wird das sterile Lyophilisat zunächst mit einer 0,9 %igen Kochsalzlösung rekonstituiert, um eine Lösung mit 25 mg/ml Pemetrexed zu erhalten, die mit der empfohlenen Dosis von 500 mg/qm Körperoberfläche, und nach weiterer Verdünnung mit 0,9 %iger Kochsalzlösung auf 100 ml verdünnt, innerhalb 10 Minuten intravenös verabreicht wird. Die chemische und physikalische Stabilität der durch Rekonstitution des Lyophilisates erhaltenen Pemetrexed-Lösung und der daraus hergestellten Infusionslösung wird angegeben mit nur 24 Stunden.

Im Hinblick auf eine sicherere Handhabung des Zytostatikums Pemetrexed war es wünschenswert, Pemetrexed in Form einer Lösung auf den Markt bringen zu können. In diesem Sinne hatte EP 2 854 765 eine flüssige pharmazeutische Lösung zur parenteralen Verabreichung vorgeschlagen, die ein Lösungsmittel, Pemetrexed oder ein pharmazeutisch annehmbares Salz davon sowie Acetylcystein oder 2-Mercaptoethansulfonat-Natrium als Antioxidationsmittel enthält.

Aufgabe der vorliegenden Erfindung ist es, eine Verwendung einer flüssigen pharmazeutischen Konzentratlösung, umfassend
- ein Lösungsmittel;
- Pemetrexed oder ein pharmazeutisch annehmbares Salz davon; sowie
- Acetylcystein und/oder 2-Mercaptoethansulfonat-Natrium als ein erstes Antioxidationsmittel,
mit einer relativ hohen Gebrauchswertigkeit für den Anwender bereitzustellen.

Diese Aufgabe wird ausgehend von einer Konzentratlösung der vorgenannten Art dadurch gelöst, dass die Konzentratlösung zur Herstellung einer Infusionslösung mittels einer Verdünnungslösung verwendet wird, wobei die Infusionslösung nach ihrer Herstellung für einen Zeitraum von länger als 72 Stunden stabil ist.

Überraschenderweise wurde festgestellt, dass eine Infusionslösung, die unter Verwendung einer flüssigen pharmazeutischen Konzentratlösung, umfassend
- ein Lösungsmittel
- Pemetrexed oder ein pharmazeutisch annehmbares Salz davon; sowie
- Acetylcystein und/oder 2-Mercaptoethansulfonat-Natrium als ein erstes Antioxidationsmittel,
mittels einer Verdünnungslösung hergestellt worden ist, verhältnismäßig stabil ist. Aus dieser verhältnismäßig hohen Stabilität ergibt sich eine erhöhte Gebrauchswertigkeit für den Anwender. So können beispielsweise aufgrund der erhöhten Stabilität der Infusionslösung und der damit einhergehenden längeren Lagerfähigkeit derselben größere Ansätze von Pemetrexed-Infusionslösungen hergestellt werden, ohne Teile davon verwerfen zu müssen, was Kosten reduziert.

Unter dem Begriff "Stabilität" wird in diesem Zusammenhang die von der Arbeitsgemeinschaft für pharmazeutische Verfahrenstechnik (APV) erarbeitete Definition verstanden, nach welcher "Stabilität" die spezifikationsgerechte Qualität des Arzneimittels bis zum Ende der vom Hersteller festgelegten Laufzeit bedeutet. Die Qualität des Arzneimittels wird dabei durch den Wirkstoffgehalt und die Reinheit, die sensorisch wahrnehmbaren, physikalisch-chemischen und die mikrobiologischen Eigenschaften bestimmt, wobei der Wirkstoffgehalt im Rahmen der vorliegenden Erfindung bis zum Ende der Haltbarkeit der Infusionslösung 95,0 % des nominellen Wertes nicht unterschreiten darf.

Entsprechend einer bevorzugten Ausführungsform der erfindungsgemäßen Verwendung ist es vorgesehen, dass die Infusionslösung nach ihrer Herstellung für einen Zeitraum von länger als 72 Stunden und bis zu 100 Tagen stabil ist, mehr bevorzugt für einen Zeitraum von länger als 72 Stunden und bis zu 80 Tagen, weiter bevorzugt für einen Zeitraum von länger als 72 Stunden und bis zu 50 Tagen, weiter bevorzugt für einen Zeitraum von länger als 72 Stunden und bis zu 35 Tagen, weiter bevorzugt für einen Zeitraum von länger als 72 Stunden und bis zu 28 Tagen, weiter bevorzugt für einen Zeitraum von länger als 72 Stunden und bis zu 21 Tagen, weiter bevorzugt für einen Zeitraum von länger als 72 Stunden und bis zu 14 Tagen und noch weiter bevorzugt für einen Zeitraum von länger als 72 Stunden und bis zu 7 Tagen.

Nach einer weiter bevorzugten Ausführungsform der erfindungsgemäßen Verwendung ist es vorgesehen, dass die Infusionslösung nach ihrer Herstellung für einen Zeitraum von länger als 84 Stunden und bis zu 100 Tagen stabil ist, mehr bevorzugt für einen Zeitraum von länger als 84 Stunden und bis zu 80 Tagen, weiter bevorzugt für einen Zeitraum von länger als 84 Stunden und bis zu 50 Tagen, weiter bevorzugt für einen Zeitraum von länger als 84 Stunden und bis zu 35 Tagen, weiter bevorzugt für einen Zeitraum von länger als 84 Stunden und bis zu 28 Tagen, weiter bevorzugt für einen Zeitraum von länger als 84 Stunden und bis zu 21 Tagen, weiter bevorzugt für einen Zeitraum von länger als 84 Stunden und bis zu 14 Tagen und noch weiter bevorzugt für einen Zeitraum von länger als 84 Stunden und bis zu 7 Tagen.

Gemäß einer weiter bevorzugten Ausführungsform der erfindungsgemäßen Verwendung ist es vorgesehen, dass die Infusionslösung nach ihrer Herstellung für einen Zeitraum von länger als 96 Stunden und bis zu 100 Tagen stabil ist, mehr bevorzugt für einen Zeitraum von länger als 96 Stunden und bis zu 80 Tagen, weiter bevorzugt für einen Zeitraum von länger als 96 Stunden und bis zu 50 Tagen, weiter bevorzugt für einen Zeitraum von länger als 96 Stunden und bis zu 35 Tagen, weiter bevorzugt für einen Zeitraum von länger als 96 Stunden und bis zu 28 Tagen, weiter bevorzugt für einen Zeitraum von länger als 96 Stunden und bis zu 21 Tagen, weiter bevorzugt für einen Zeitraum von länger als 96 Stunden und bis zu 14 Tagen und noch weiter bevorzugt für einen Zeitraum von länger als 96 Stunden und bis zu 7 Tagen.

Entsprechend einer weiter bevorzugten Ausführungsform der erfindungsgemäßen Verwendung ist es vorgesehen, dass die Infusionslösung nach ihrer Herstellung bei einer Temperatur von 2 °C bis 8 °C für die vorgenannten Zeiträume stabil ist, optional bei einer relativen Luftfeuchtigkeit (bei Lagerung) von 60 % und/oder optional unter Ausschluss von Licht.

Entsprechend einer weiter bevorzugten Ausführungsform der erfindungsgemäßen Verwendung ist es vorgesehen, dass die Verdünnungslösung eine Natriumchlorid- oder eine Glukoselösung ist. In diesem Zusammenhang ist es bevorzugt, wenn die Natriumchloridlösung eine 0,9 %ige Natriumchloridlösung mit dem Lösungsmittel Wasser ist oder wenn die Glukoselösung eine 5 %ige Glukoselösung mit dem Lösungsmittel Wasser ist. Erfindungsgemäß besonders bevorzugt ist es in dieser Hinsicht, dass die Verdünnungslösung eine Natriumchloridlösung ist, insbesondere eine 0,9 %ige Natriumchloridlösung mit dem Lösungsmittel Wasser.

Nach einer weiter bevorzugten Ausführungsform der erfindungsgemäßen Verwendung ist es vorgesehen, dass die Verdünnungslösung ein zweites Antioxidationsmittel enthält, wobei es in diesem Zusammenhang besonders bevorzugt ist, wenn das zweite Antioxidationsmittel gleich dem ersten Antioxidationsmittel der Pemetrexed-Konzentratlösung ist. Ist in der Verdünnungslösung ein zweites Antioxidationsmittel enthalten, kann die Stabilität der resultierenden Infusionslösung weiter verbessert werden.

Gemäß einer weiter bevorzugten Ausführungsform der erfindungsgemäßen Verwendung ist es vorgesehen, dass das Lösungsmittel der Pemetrexed-Konzentratlösung ausgewählt ist aus der Gruppe bestehend aus Wasser, Polyethylenglycol und Ethanol sowie aus Mischungen von zwei oder mehr der genannten Lösungsmittel. Es konnte festgestellt werden, dass die genannten Lösungsmittel bzw. Lösungsmittelgemische gute Lösungseigenschaften für Wirkstoff und erstes Antioxidans aufweisen, die Stabilität des Wirkstoffs Pemetrexed nicht nachteilig beeinflussen und eine Partikelbildung in der Konzentratlösung nicht fördern.

Erfindungsgemäß besonders bevorzugt ist es, wenn das Lösungsmittel der Konzentratlösung Wasser ist.

Entsprechend einer weiteren bevorzugten Ausführungsform der erfindungsgemäßen Verwendung ist es vorgesehen, dass das Pemetrexed in der Konzentratlösung in Form seines Dinatriumsalzes gelöst enthalten ist. Das Pemetrexed-Dinatriumsalz weist verhältnismäßig gute Löslichkeitseigenschaften auf. So können mittels des Pemetrexed-Dinatriumsalzes beispielsweise Pemetrexed-Konzentratlösungen mit einer relativ hohen Pemetrexedkonzentration bereitgestellt werden, die eine hohe Stabilität des Wirkstoffs zeigen und nicht zur Eintrübung oder zur Ausbildung von Niederschlag neigen. Derartige Konzentrate besitzen eine hohe Akzeptanz beim Patienten und können vom medizinischen Personal einfach und sicher gehandhabt werden.

Nach einer weiteren bevorzugten Ausführungsform der erfindungsgemäßen Verwendung ist es vorgesehen, dass der Gehalt der Konzentratlösung an Pemetrexed bzw. an pharmazeutisch annehmbaren Salz davon (bezogen auf das freie Pemetrexed) 0,1 mg/ml bis 100 mg/ml beträgt, mehr bevorzugt 5 mg/ml bis 80 mg/ml, noch mehr bevorzugt 10 mg/ml bis 50 mg/ml und weiter bevorzugt 20 mg/ml bis 40 mg/ml. In den genannten Konzentrationsintervallen zeigt die Konzentratlösung eine nur relativ geringe Tendenz zur Partikelbildung und eine damit verbundene Eintrübung der Lösung oder Ausbildung von Niederschlag.

Gemäß einer weiteren bevorzugten Ausführungsform der erfindungsgemäßen Verwendung ist es vorgesehen, dass der Gehalt der Konzentratlösung an Pemetrexed bzw. an pharmazeutisch annehmbaren Salz davon (bezogen auf das freie Pemetrexed) 10 mg/ml, 20 mg/ml, 25 mg/ml, 40 mg/ml oder 50 mg/ml beträgt.

Nach einer weiteren bevorzugten Ausführungsform der erfindungsgemäßen Verwendung ist es vorgesehen, dass der Gehalt der Infusionslösung an Pemetrexed bzw. an pharmazeutisch annehmbaren Salz davon (bezogen auf das freie Pemetrexed) 2 mg/ml bis 15 mg/ml beträgt, vorzugsweise 3 mg/ml bis 13 mg/ml. In den genannten Konzentrationsintervallen zeigt die Infusionslösung eine verhältnismäßig hohe Stabilität.

Entsprechend einer weiteren bevorzugten Ausführungsform der erfindungsgemäßen Verwendung ist es vorgesehen, dass das Antioxidationsmittel Acetylcystein ist. Es konnte festgestellt werden, dass Acetylcystein (oder anders N-Acetyl-L-cystein) in der Konzentratlösung und damit letztlich auch in der Infusionslösung eine verhältnismäßig hohe Stabilität des Pemetrexeds bewirkt und dass eine entsprechende Konzentratlösung/Infusionslösung verhältnismäßig lange Zeit gelagert werden kann, ohne dass sie eintrübt oder aus ihr gar ein Niederschlag ausfällt bzw. die Wirkstoffkonzentration unterhalb des geforderten Wertes des Stabilitätskriteriums fällt.

Nach einer weiteren bevorzugten Ausführungsform der erfindungsgemäßen Verwendung ist es vorgesehen, dass wenn Acetylcystein als Antioxidationsmittel in der Konzentratlösung enthalten ist, die Konzentratlösung und die Infusionslösung einen pH-Wert in einem Bereich von 7,5 bis 11,5 aufweisen, mehr bevorzugt einen pH-Wert in einem Bereich von 8,0 bis 10,5 und noch mehr bevorzugt einen pH-Wert in einem Bereich von 8,2 bis 10,5. Es wurde festgestellt, dass der Wirkstoff in den genannten pH-Wertintervallen durch eine verhältnismäßig hohe Stabilität insbesondere gegenüber oxidativem Abbau gekennzeichnet ist, und zwar sowohl in der Konzentrat- als auch in der Infusionslösung.

Nach einer weiter bevorzugten Ausführungsform der erfindungsgemäßen Verwendung ist es vorgesehen, dass das Antioxidationsmittel 2-Mercaptoethansulfonat-Natrium ist. Es konnte festgestellt werden, dass 2-Mercaptoethansulfonat-Natrium in der Konzentratlösung und in der Infusionslösung eine verhältnismäßig hohe Stabilität des Pemetrexeds bewirkt und dass eine entsprechende Konzentratlösung und eine daraus resultierende Infusionslösung entsprechend verhältnismäßig lange Zeit gelagert werden können.

Gemäß einer weiteren bevorzugten Ausführungsform der erfindungsgemäßen Verwendung ist es vorgesehen, dass wenn 2-Mercaptoethansulfonat-Natrium als Antioxidationsmittel in der Konzentratlösung enthalten ist, die Konzentratlösung und die Infusionslösung einen pH-Wert in einem Bereich von 7,5 bis 11,5 aufweisen, mehr bevorzugt einen pH-Wert in einem Bereich von 8,0 bis 10,5, noch mehr bevorzugt einen pH-Wert in einem Bereich von 8,0 bis 10,0 und weiter bevorzugt einen pH-Wert in einem Bereich von 8,3 bis 9,3. Es wurde festgestellt, dass der Wirkstoff in der Konzentrat- und in der Infusionslösung in den genannten pH-Wertintervallen durch eine verhältnismäßig hohe Stabilität insbesondere gegenüber oxidativem Abbau gekennzeichnet ist.

Entsprechend einer weiteren bevorzugten Ausführungsform der erfindungsgemäßen Verwendung ist es vorgesehen, dass der Gehalt der Konzentratlösung an Antioxidationsmittel 0,1 mg/ml bis 100 mg/ml beträgt, mehr bevorzugt 0,5 mg/ml bis 20 mg/ml, noch mehr bevorzugt 1,0 mg/ml bis 5 mg/ml und weiter bevorzugt 1 mg/ml bis 3 mg/ml. Es konnte festgestellt werden, dass der Wirkstoff in der Konzentratlösung und in der Infusionslösung hinsichtlich genannter Konzentrationsintervalle der Konzentratlösung durch eine verhältnismäßig hohe Stabilität insbesondere gegenüber oxidativem Abbau gekennzeichnet ist.

Nach einer weiteren bevorzugten Ausführungsform der erfindungsgemäßen Verwendung ist es vorgesehen, dass die Konzentratlösung ferner einen oder mehrere pharmazeutische Hilfsstoffe umfasst, ausgewählt aus der Gruppe bestehend aus Salzen, Kohlehydraten zur Tonisierung, Chelatbildnern zur Komplexierung von Schwermetallen, Säuren zur pH-Wert Einstellung, Basen zur pH-Wert Einstellung, Puffersubstanzen und Konservierungsmitteln zur mikrobiellen Konservierung der Lösung. Die genannten Substanzen sind dem zuständigen Fachmann aus dem Stand der Technik hinlänglich bekannt.

Entsprechend einer weiter bevorzugten Ausführungsform der erfindungsgemäßen Verwendung ist es vorgesehen, dass die Konzentratlösung Mannitol zur Tonisierung enthält.

Gemäß einer weiter bevorzugten Ausführungsform der erfindungsgemäßen Verwendung ist es vorgesehen, dass die Verdünnungslösung ein antimikrobielles Konservierungsmittel enthält. Entsprechend enthält dann auch die resultierende Infusionslösung das antimikrobielle Konservierungsmittel, weshalb eine längeren Lagerung der angesetzten Infusionslösung dann auch aus mikrobieller Hinsicht unproblematisch ist.

Ein typischer Gegenstand, der durch die vorliegende Erfindung bereitgestellt wird, betrifft die Verwendung einer flüssigen pharmazeutischen Konzentratlösung, umfassend
- Wasser als Lösungsmittel;
- Pemetrexed oder ein pharmazeutisch annehmbares Salz davon, insbesondere Pemetrexed-Dinatrium; sowie
- Acetylcystein als ein erstes Antioxidationsmittel,
zur Herstellung einer Infusionslösung mittels einer Verdünnungslösung, insbesondere einer Natriumchloridlösung als Verdünnungslösung, wobei die Infusionslösung nach ihrer Herstellung für einen Zeitraum von länger als 72 Stunden oder 84 Stunden oder 96 Stunden stabil ist, insbesondere für einen Zeitraum von länger als 72 Stunden oder 84 Stunden oder 96 Stunden und bis zu 100 Tagen oder 50 Tagen oder 35 Tagen.

Ein weiterer typischer Gegenstand, der durch die vorliegende Erfindung bereitgestellt wird, betrifft die Verwendung einer flüssigen pharmazeutischen Konzentratlösung, umfassend
- Wasser als Lösungsmittel;
- Pemetrexed oder ein pharmazeutisch annehmbares Salz davon, insbesondere Pemetrexed-Dinatrium; sowie
- Acetylcystein als ein erstes Antioxidationsmittel,
zur Herstellung einer Infusionslösung mittels einer Natriumchloridlösung als Verdünnungslösung, wobei die Infusionslösung nach ihrer Herstellung für einen Zeitraum von länger als 84 Stunden oder 96 Stunden stabil ist, insbesondere für einen Zeitraum von länger als 84 Stunden oder 96 Stunden bis zu 100 Tagen oder 50 Tagen oder 35 Tagen.

Die nachstehenden Beispiele dienen der Erläuterung der Erfindung.

### Beispiel 1:

Herstellung einer Pemetrexed-Konzentratlösung mit einer Konzentration von 25 mg/ml Pemetrexed für eine 500 mg Infusionslösung
1 ml der Pemetrexed-Konzentratlösung enthält:

| Pemetrexed-Dinatrium | 27,57 mg (i.e. 25 mg freies Pemetrexed) |
|---|---|
| Mannitol | 25,00 mg |
| Acetylcystein | 3,00 mg |
| Natriumhydroxid | 0,818 mg-0,854 mg |
| Hydrogenchlorid | 0 mg-0,032 mg |
| Wasser für Injektionszwecke | 963,58 mg |
| Stickstoff | |
| Lösungsdichte | 1,020 g/ml |

Für die Herstellung der Zusammensetzung gemäß Beispiel 1 im 30 Liter Ansatz wurden 90 % der benötigten Menge Wasser für Injektionszwecke (20 °C bis 25 °C) im Ansatzkessel vorgelegt und anschließend mindestens 10 Minuten mit Stickstoff begast, bis der Sauerstoffgehalt bei < 0,5 mg/l lag.

Die Gesamtmenge an Acetylcystein wurde unter Rühren in die Vorlage eingebracht und bis zur vollständigen Auflösung gerührt. Danach wurde die Gesamtmenge an Mannitol unter Rühren in die Vorlage eingebracht und bis zur vollständigen Auflösung gerührt. Der pH-Wert wurde mit 10 %iger (w/w) Natriumhydroxidlösung und ggfs. mit 10 %iger (w/w) Salzsäure auf einen pH-Wert von 9,0 +/- 0,1 eingestellt.

Danach wurde die Gesamtmenge an Pemetrexed-Dinatrium unter Rühren in die so erhaltene Lösung eingebracht und bis zur vollständigen Auflösung gerührt. Der pH-Wert wurde dann mit 10 %iger (w/w) Natriumhydroxid-lösung und ggfs. mit 10 %iger (w/w) Salzsäure auf einen pH-Wert von 9,0 +/- 0,1 eingestellt.

Der so erhaltene Ansatz wurde mit Wasser für Injektionszwecke auf das gewünschte Endgewicht/-volumen aufgefüllt und bis zur vollständigen Lösung gerührt. Danach wurde der pH-Wert mit 10 %iger (w/w) Natriumhydroxidlösung und ggfs. mit 10 %iger (w/w) Salzsäure auf pH 9,0 +/- 0,1 eingestellt.

Der so erhaltene Ansatz wurde dann mindestens 10 Minuten mit Stickstoff begast, bis der Sauerstoffgehalt bei < 0,5 mg/l lag.

Die so erhaltene Ansatzlösung wurde im Ansatzkessel mit Stickstoff überlagert und dicht verschlossen. Nach Entnahme der Inprozesskontrollmuster erfolgte die Sterilfiltration der Lösung (20 °C bis 25 °C) über sterilisierte Filtermembrancapsulen mit 0,2 µm Porenweite unter aseptischen Bedingungen in einem sterilen, mit sterilem Stickstoff vorgespülten Lagertank. Die Abfüllung der Lösung (20 °C bis 25 °C) erfolgte unter aseptischen Bedingungen zu jeweils 20,50 ml in hitzesterilisierten 20R DIN-Vials (Fiolaxglas; 500 mg).

Während der Abfüllung erfolgte eine Vor-, Füll- und Nachbegasung der Vial mit sterilem Stickstoff. Nach Aufsetzen der Hohlstopfen und Verbördelung der Vial erfolgte nach Außenwaschung, Trocknung und Dichtigkeitsprüfung (Überkopflagerung; 12 Stunden) eine terminale Sterilisation im Endbehältnis (121 °C, 20 min) vor deren Einlagerung bei 2 °C bis 8 °C.

### Beispiel 2:

Es wurden Infusionslösungen mit einer Konzentration von 3,5 mg/ml, 7,0 mg/ml bzw. 12,5 mg/ml Pemetrexed (berechnet als freies Pemetrexed; liegt in der Infusionslösung als Pemetrexed-Dinatrium vor) hergestellt. Dabei wurden für jede der genannten Pemetrexed-Konzentrationen jeweils insgesamt sechs Infusionsbeutel mit Infusionslösung präpariert, und zwar für jeden Messpunkt der Einlagerungsdauer einen (vgl. Tabellen in Beispiel 3).

Zur Präparation der Infusionslösungen wurden zunächst handelsübliche, mit 100 ml einer 0,9 %igen wässrigen NaCl-Lösung als Verdünnungslösung befüllte Infusionskunststoffbeutel vorgelegt. Diesen wurden in Abhängigkeit von der einzustellenden Konzentration 14 ml, 28 ml bzw. 50 ml der NaCl-Lösung entnommen und den Beuteln danach 14 ml, 28 ml bzw. 50 ml der Pemetrexed-Konzentratlösung gemäß Beispiel 1 zugesetzt. Dabei war die Pemetrexed-Konzentratlösung gemäß Beispiel 1 zuvor über einen Zeitraum von 3,5 Jahren bei einer Temperatur von 2 °C bis 8 °C eingelagert worden.

Die so präparierten Infusionslösungen/Infusionsbeutel wurden bei einer Temperatur von 2 °C bis 8 °C unter Lichtausschluss eingelagert.

### Beispiel 3:

Die gemäß Beispiel 2 präparierten Infusionslösungen wurden nach der jeweiligen Lagerdauer in Anlehnung an die Monographie Nr. 10.0/2637 (Titel: "Pemetrexed-Dinatrium-Pentahydrat") des Europäischen Arzneibuchs Ph. Eur. 10. Ausgabe, Grundwerk 2020, Band 4, Seiten 5214 bis 5217 mittels HPLC auf ihren Pemetrexed-Gehalt hin sowie auf ihren Gehalt an Pemetrexed-Verunreinigungen hin untersucht (die unten in den Tabellen 1 bis 3 angegeben Pemetrexed-Verunreinigungen A bis D sind in vorgenannter Monographie chemisch spezifiziert).

### Die Parameter zur Bestimmung des Pemetrexed-Gehalts der Infusionslösungen mittels HPLC waren wie folgt:

Pemetrexed wurde mittels einer Phasensäule RP-C8 separiert und mittels UV-Detektion bei 285 nm direkt quantifiziert.

### Referenzsubstanz:

### Pemetrexed-Dinatrium-Heptahydrat CRS

### Lösungen:

Referenzlösung: 30,0 mg (bezogen auf freies Pemetrexed) Pemetrexed-Dinatrium-Heptahydrat CRS wurden in Wasser zur Chromatographie R zu 200,0 ml gelöst.
Acetatpuffer: 1,7 ml Essigsäure 99 % R und 900 ml Wasser zur Chromatographie R wurden gemischt. Die Lösung wurde mit einer Lösung von Natriumhydroxid R (760 g l⁻¹) in Wasser zur Chromatographie R auf einen pH-Wert von 5,3 eingestellt und mit Wasser zur Chromatographie R zu 1000 ml verdünnt.
Mobile Phase: Acetonitril R, Acetatpuffer (11:89 V/V).
Probenlösungen: die Infusionslösungen wurden vor ihrer HPLC-Vermessung mittels Wasser für HPLC R auf eine nominelle Pemetrexed-Konzentration von 0,15 mg/ml verdünnt.

### Chromatographie-System:

Gerät: HPLC Ultimate 3000 von Thermo Fisher Scientific, ausgestattet mit einem Diodenarraydetektor und einem Säulenofen.
Säule: Zorbax SB C8, 3,5 *µ*m, 150 x 4,6 mm, der Firma Agilent Technologies, Artikelnummer 863953-906.
Mobile Phase: Acetonitril R, Acetatpuffer (11:89 V/V).
Durchflussrate: 2,0 ml/min
Injektionsvolumen: 20 *µ*l
Detektion: Spektrometer bei 285 nm
Probensammler-Temperatur: 5 °C
Ofentemperatur: 30 °C
Stoppzeit: 10 min

### Die Parameter zur Bestimmung des Pemetrexedverunreinigungs-Gehalts der Infusionslösungen mittels HPLC waren wie folgt:

Pemetrexed-Verunreinigungen wurden mittels einer Phasensäule RP-C8 separiert und mittels UV-Detektion bei 250 nm direkt quantifiziert.

### Referenzsubstanzen:

### Pemetrexed-Dinatrium-Heptahydrat CRS

### Durchstechflasche mit Pemetrexed-Verunreinigungsmischung CRS (Verunreinigungen A und D)

### Lösungen:

Lösung A: Lösung von Ammoniumformiat R (1,45 g l⁻¹) in Wasser zur Chromatographie R, mit wasserfreier Ameisensäure R auf einen pH-Wert von 3,5 eingestellt.
Referenzlösung a: 20 mg Pemetrexed-Dinatrium-Heptahydrat CRS (bezogen auf freies Pemetrexed) wurden in Wasser zur Chromatographie R zu 100,0 ml gelöst. 1,0 ml davon wurde mit Wasser zur Chromatographie R zu 100,0 ml verdünnt. 1,0 ml dieser Lösung wurde mit Wasser zur Chromatographie R zu 10,0 ml verdünnt.
Referenzlösung b: Zur Herstellung der Verunreinigungen B und C *in* situ wurden 30 mg Pemetrexed-Dinatrium-Heptahydrat CRS (bezogen auf freies Pemetrexed) in 10,0 ml einer Lösung von Natriumhydroxid R (4,0 g l⁻¹) gelöst. Die Lösung wurde 40 min lang bei 70 °C erhitzt und anschließend erkalten gelassen. 1,0 ml dieser Lösung wurde mit Wasser zur Chromatographie R zu 10,0 ml verdünnt.
Referenzlösung c: Der Inhalt einer Durchstechflasche mit der Pemetrexed-Verunreinigungsmischung CRS (Verunreinigungen A und D) wurde in 1,0 ml Wasser zur Chromatographie R gelöst.
Identifizierung von Verunreinigungen: Zur Identifizierung der Peaks der Verunreinigungen A und D wurden das mitgelieferte Chromatogramm der Pemetrexed-Verunreinigungsmischung CRS und das mit der Referenzlösung c erhaltene Chromatogramm verwendet. Zur Identifizierung der Peaks der Verunreinigungen B und C wurde das mit der Referenzlösung b erhaltene Chromatogramm verwendet.
Mobile Phase A: Acetonitril R, Lösung A (5:95 V/V).
   Mobile Phase B: Acetonitril R, Lösung A (30:70 V/V)
Probenlösungen: die Infusionslösungen wurden vor ihrer HPLC-Vermessung mittels Wasser für HPLC R auf eine nominelle Pemetrexed-Konzentration von 0,2 mg/ml verdünnt.
Für die Berechnung der Prozentgehalte für jede Verunreinigung wurde die Konzentration von Pemetrexed-Dinatrium-Heptahydrat in der Referenzlösung a verwendet.

### Chromatographie-System:

Gerät: HPLC Ultimate 3000 von Thermo Fisher Scientific, ausgestattet mit einem Diodenarraydetektor und einem Säulenofen.
Säule: Zorbax SB C8, 3,5 *µ*m, 150 x 4,6 mm, der Firma Agilent Technologies, Artikelnummer 863953-906.
Mobile Phase A: Acetonitril R, Lösung A (5:95 V/V).
   Mobile Phase B: Acetonitril R, Lösung A (30:70 V/V)
Durchflussrate: 1,0 ml/min
Injektionsvolumen: 20 *µ*l
Detektion: Spektrometer bei 250 nm
Probensammler-Temperatur: 5 °C
Ofentemperatur: 25 °C
Stoppzeit: 50 min

### Gradient:

| Zeit (min) | Mobile Phase A (% v/v) | Mobile Phase B (% v/v) |
|---|---|---|
| 0-3 | 100 | 0 |
| 3-45 | 100 → 0 | 0 → 100 |
| 45-50 | 0 → 100 | 100 → 0 |

Die für die Infusionslösungen mit einem nominellen Pemetrexed-Gehalt von 3,5 mg/ml, 7 mg/ml und 12,5 mg/ml aus den HPLC-Messungen erhaltenen Werte des Pemetrexed-Gehalts und der Gehalte an Pemetrexed-Verunreinigungen sind in den Tabellen 1, 2 bzw. 3 wiedergegeben.

**Tabelle 1: (3,5 mg/ml)**

| Lagerdauer | 0 Tage | 7 Tage | 14 Tage | 21 Tage | 28 Tage | 35 Tage |
|---|---|---|---|---|---|---|
| Pemetrexed | 100% | 100% | 101% | 100% | 100% | 102% |
| Verunreinigung A | 0,06% | 0,05% | 0,06% | 0,06% | 0,05% | 0,05% |
| Verunreinigung B | <0,05% | <0,05% | <0,05% | <0,05% | <0,05% | <0,05% |
| Verunreinigung C | <0,05% | <0,05% | <0,05% | <0,05% | <0,05% | <0,05% |
| Verunreinigung D | <0,05% | <0,05% | <0,05% | <0,05% | <0,05% | <0,05% |
| Summe andere Verunreinigungen | 0,48% | 0,47% | 0,49% | 0,49% | 0,47% | 0,49% |
| Summe der Verunreinigungen | 0,59% | 0,52% | 0,55% | 0,60% | 0,52% | 0,54% |

**Tabelle 2: (7 mg/ml)**

| Lagerdauer | 0 Tage | 7 Tage | 14 Tage | 21 Tage | 28 Tage | 35 Tage |
|---|---|---|---|---|---|---|
| Pemetrexed | 100% | 98% | 97% | 96% | 98% | 98% |
| Verunreinigung A | 0,06% | 0,06% | 0,05% | 0,06% | 0,05% | 0,06% |
| Verunreinigung B | <0,05% | <0,05% | <0,05% | <0,05% | <0,05% | <0,05% |
| Verunreinigung C | <0,05% | <0,05% | <0,05% | <0,05% | <0,05% | <0,05% |
| Verunreinigung D | <0,05% | <0,05% | <0,05% | <0,05% | <0,05% | <0,05% |
| Summe andere Verunreinigungen | 0,48% | 0,50% | 0,50% | 0,51% | 0,49% | 0,49% |
| Summe der Verunreinigungen | 0,59% | 0,56% | 0,55% | 0,57% | 0,54% | 0,55% |

**Tabelle 3: (12,5 mg/ml)**

| Lagerdauer | 0 Tage | 7 Tage | 14 Tage | 21 Tage | 28 Tage | 35 Tage |
|---|---|---|---|---|---|---|
| Pemetrexed | 100% | 101% | 103% | 102% | 102% | 101% |
| Verunreinigung A | 0,06% | 0,06% | 0,06% | 0,06% | 0,06% | 0,06% |
| Verunreinigung B | <0,05% | <0,05% | <0,05% | <0,05% | <0,05% | <0,05% |
| Verunreinigung C | <0,05% | <0,05% | <0,05% | <0,05% | <0,05% | <0,05% |
| Verunreinigung D | <0,05% | <0,05% | <0,05% | <0,05% | <0,05% | <0,05% |
| Summe andere Verunreinigungen | 0,48% | 0,53% | 0,57% | 0,57% | 0,55% | 0,57% |
| Summe der Verunreinigungen | 0,59% | 0,59% | 0,63% | 0,63% | 0,61% | 0,62% |

## Patentansprüche

1. Verwendung einer flüssigen pharmazeutische Konzentratlösung, umfassend
- ein Lösungsmittel;
- Pemetrexed oder ein pharmazeutisch annehmbares Salz davon; sowie
- Acetylcystein und/oder 2-Mercaptoethansulfonat-Natrium als ein erstes Antioxidationsmittel,
zur Herstellung einer Infusionslösung mittels einer Verdünnungslösung, wobei die Infusionslösung nach ihrer Herstellung für einen Zeitraum von länger als 72 Stunden stabil ist.

2. Verwendung nach Anspruch 1, **dadurch gekennzeichnet, dass** die Infusionslösung nach ihrer Herstellung für einen Zeitraum von länger als 72 Stunden und bis zu 100 Tagen stabil ist.

3. Verwendung nach einem der voranstehenden Ansprüche, **dadurch gekennzeichnet, dass** die Infusionslösung nach ihrer Herstellung bei einer Temperatur von 2 °C bis 8 °C für den Zeitraum stabil ist.

4. Verwendung nach einem der voranstehenden Ansprüche, **dadurch gekennzeichnet, dass** die Verdünnungslösung eine Natriumchlorid- oder eine Glukoselösung ist.

5. Verwendung nach einem der voranstehenden Ansprüche, **dadurch gekennzeichnet, dass** die Verdünnungslösung ein zweites Antioxidationsmittel enthält.

6. Verwendung nach einem der voranstehenden Ansprüche, **dadurch gekennzeichnet, dass** das Lösungsmittel der Konzentratlösung ausgewählt ist aus der Gruppe bestehend aus Wasser, Polyethylenglycol und Ethanol sowie aus Mischungen von zwei oder mehr der genannten Lösungsmittel.

7. Verwendung nach einem der voranstehenden Ansprüche, **dadurch gekennzeichnet, dass** das Pemetrexed in der Konzentratlösung in Form seines Dinatriumsalzes gelöst enthalten ist.

8. Verwendung nach einem der voranstehenden Ansprüche, **dadurch gekennzeichnet, dass** der Gehalt der Konzentratlösung an Pemetrexed bzw. an pharmazeutisch annehmbaren Salz davon 20 mg/ml bis 40 mg/ml beträgt.

9. Verwendung nach einem der voranstehenden Ansprüche, **dadurch gekennzeichnet, dass** der Gehalt der Konzentratlösung an Pemetrexed bzw. an pharmazeutisch annehmbaren Salz davon 20 mg/ml, 25 mg/ml oder 40 mg/ml beträgt.

10. Verwendung nach einem der voranstehenden Ansprüche, **dadurch gekennzeichnet, dass** der Gehalt der Infusionslösung an Pemetrexed bzw. an pharmazeutisch annehmbaren Salz davon 2 mg/ml bis 15 mg/ml beträgt, vorzugsweise 3 mg/ml bis 13 mg/ml.

11. Verwendung nach einem der voranstehenden Ansprüche, **dadurch gekennzeichnet, dass** das erste Antioxidationsmittel Acetylcystein ist.

12. Verwendung nach einem der voranstehenden Ansprüche, **dadurch gekennzeichnet, dass** die Infusionslösung einen pH-Wert in einem Bereich von 7,5 bis 11,5 aufweist, mehr bevorzugt einen pH-Wert in einem Bereich von 8,0 bis 10,5 und noch mehr bevorzugt einen pH-Wert in einem Bereich von 8,2 bis 10,5.

13. Verwendung nach einem der voranstehenden Ansprüche, **dadurch gekennzeichnet, dass** der Gehalt der Konzentratlösung an erstem Antioxidationsmittel 0,1 mg/ml bis 100 mg/ml beträgt, mehr bevorzugt 0,5 mg/ml bis 20 mg/ml, noch mehr bevorzugt 1,0 mg/ml bis 5 mg/ml und weiter bevorzugt 1 mg/ml bis 3 mg/ml.

14. Verwendung nach einem der voranstehenden Ansprüche, **dadurch gekennzeichnet, dass** die Konzentratlösung ferner einen oder mehrere pharmazeutische Hilfsstoffe umfasst, ausgewählt aus der Gruppe bestehend aus Salzen, Kohlehydraten zur Tonisierung, Chelatbildnern zur Komplexierung von Schwermetallen, Säuren zur pH-Wert Einstellung, Basen zur pH-Wert Einstellung, Puffersubstanzen, Konservierungsmitteln zur mikrobiellen Konservierung der Lösung.

15. Verwendung nach einem der voranstehenden Ansprüche, **dadurch gekennzeichnet, dass** die Konzentratlösung Mannitol zur Tonisierung enthält.
